# EUROPEAN PATENT APPLICATION

(11) **EP 3 805 204 A1**
(43) Date of publication of application: **14.04.2021**
(21) Application number: 19807109.4
(22) Date of filing: 23.05.2019
(51) Int. Cl.: C07D 213/81, A61K 31/44, A61P 9/10, A61P 7/06

(54) **METHOD FOR PREPARING ALKYNYL PYRIDINE PROLYL HYDROXYLASE INHIBITOR**

(30) Priority: 24.05.2018 CN 201810509984
(71) Applicant: Suzhou Suncadia Biopharmaceuticals Co., Ltd., Suzhou, Jiangsu 215126 (CN); Shanghai Shengdi Pharmaceutical Co., Ltd, Shanghai 201210 (CN); Jiangsu Hengrui Medicine Co., Ltd., Lianyungang, Jiangsu 222047 (CN)
(72) Inventor: LU, Gang, Suzhou, Jiangsu 215126 (CN); HUANG, Jian, Shanghai 201210 (CN); HU, Yimin, Suzhou, Jiangsu 215126 (CN); ZHU, Lingjian, Shanghai 201210 (CN); ZOU, Yang, Shanghai 201210 (CN); CUI, Hua, Suzhou, Jiangsu 215126 (CN); YOU, Qidong, Nanjing, Jiangsu 211198 (CN); ZHANG, Xiaojin, Nanjing, Jiangsu 211198 (CN)
(74) Representative: Potter Clarkson
(86) International application number: PCT/CN2019/088150
(87) International publication number: WO 2019/223764

(57) **Abstract**

Provided is a method for preparing an alkynyl pyridine prolyl hydroxylase inhibitor. In particular, the method involved uses 3-substituted-5-bromopyridine-2-carboxylic acid protected by different protection groups as raw materials, and by means of condensation, Sonogashira coupling and deprotection, the target compound is obtained. The process reaction conditions are simple, and have no harsh reaction conditions, strong operability, and stable amplification.

## Description

### FIELD OF THE INVENTION

The present invention relates to a method for preparing an alkynyl pyridine prolyl hydroxylase inhibitor.

### BACKGROUND OF THE INVENTION

Alkynyl pyridine prolyl hydroxylase (PHD) inhibitor is a kind of potential therapy used to treat and prevent anemia and ischemic diseases (such as anemia in chronic kidney disease, myocardial ischemia, cerebral ischemia, stroke and the like). Its mechanism is to increase the content of the alpha subunit of hypoxia inducible factor (HIF) by inhibiting PHD, thereby increasing the production and secretion of erythropoietin (EPO), promoting the maturation of red blood cells, enhancing the capacity of blood in delivering oxygen, and improving the symptoms of anemia or ischemia.

CN105130888A discloses a method for preparing such compounds, and an example is as follows:

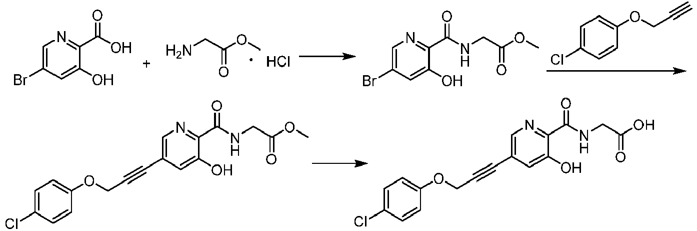

3-Hydroxy-5-bromopyridine-2-carboxylic acid as the starting material is reacted with glycine methyl ester hydrochloride to obtain an amide. The resulting amide is reacted with substituted propyne in the presence of cuprous iodide and dichlorobis(triphenylphosphine)palladium under microwave to obtain a coupling product. The resulting coupling product is subjected to a deprotection of ester group to obtain the final product. However, this process route has the following problems: 1) in the method for preparing the starting material 3-hydroxy-5-bromopyridine-2-carboxylic acid, the step of preparing 5-bromo-3-hydroxy-2-cyanopyridine from 5-bromo-3-nitro-2-cyanopyridine will violently release heat and produce a lot of impurities, which is not suitable for industrial production, and the compound with unprotected phenolic hydroxy is prone to esterification reaction of the compound itself in the next step; 2) 1-hydroxybenzotriazole (HOBT), used as the condensing agent in the amide formation step, is expensive and easy to leave a residue, thereby increasing the production cost, and anhydrous HOBT is prone to explosion and brings safety hazards; 3) the microwave reaction used in the route cannot be industrialized; 4) the yield of the final product is low; and 5) the product obtained in the condensation step or coupling step needs to be purified by silica gel column chromatography, which is not suitable for mass production.

### SUMMARY OF THE INVENTION

The present invention provides a method for preparing a compound of formula (III) characterized in that a compound of formula (IV) is reacted with a compound of formula (VI) in the presence of a palladium catalyst, copper catalyst and alkali to obtain the compound of formula (III), 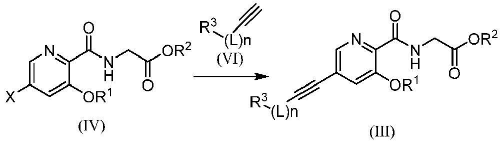
wherein R¹ is a hydroxy protecting group;
R² is a carboxy protecting group;
R³ is a C₁-C₄ alkyl, phenyl, substituted phenyl, 5 to 6 membered heteroaryl containing oxygen or nitrogen, substituted 5 to 6 membered heteroaryl containing oxygen or nitrogen, wherein the substituent is a C₁-C₄ alkyl, C₁-C₄ alkoxy, C₁-C₄ haloalkyl, halogen, cyano, 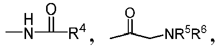 phenyl, or 5 to 6 membered heteroaryl containing oxygen or nitrogen, wherein R⁴ is a C₁-C₄ alkyl; R⁵ and R⁶ are each independently selected from the group consisting of hydrogen and C₁-C₄ alkyl, or R⁵ and R⁶ are connected to form a 3 to 7 membered heterocyclyl containing nitrogen;
L is -CH₂-, -CH₂O- or 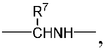 wherein R⁷ is selected from the group consisting of hydrogen, C₁-C₄ alkyl and phenyl;
n is 0 or 1; and
X is an iodine, bromine, chlorine, or triflate group.

The method for preparing the compound of formula (III) provided by the present invention is characterized in that the palladium catalyst is at least one selected from the group consisting of Pd₂(dba)₃, Pd(dba)₂, Pd(OAc)₂, Pd(tfa)₂, Pd(Piv)₂, Pd(OTf)₂, Pd(PPh₃)₄, PdCl₂, Pd(PPh₃)₂Cl₂ and Pd(dppf)Cl₂, and preferably Pd(PPh₃)₂Cl₂; the copper catalyst is at least one selected from the group consisting of CuI, CuBr, CuCl and CuF, and preferably CuI; and the alkali is at least one selected from the group consisting of triethylamine, trimethylamine and diisopropylethylamine, and preferably triethylamine.

The method for preparing the compound of formula (III) provided by the present invention is characterized in that the molar ratio of the palladium catalyst to the compound of formula (IV) is 0.001:1 to 1:1, and preferably 0.01:1 to 0.05:1; and the molar ratio of the copper catalyst to the palladium catalyst is 10:1 to 1:10, and preferably 2:1 to 1:2.

The method for preparing the compound of formula (III) provided by the present invention is characterized in that the reaction solvent is at least one selected from the group consisting of tetrahydrofuran, 2-methyltetrahydrofuran, 1,4-dioxane, N,N-dimethylformamide, N,N-dimethylacetamide, N-methylpyrrolidone, dimethyl sulfoxide, hexamethylphosphoramide and 1,3-dimethyl-2-imidazolinone, and preferably tetrahydrofuran.

The present invention provides a method for preparing a compound of formula (I) characterized by comprising the method for preparing the compound of formula (III), and a further step of removing groups R¹ and R², 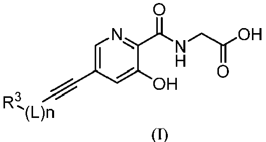 wherein R¹, R² , R³, L and n are as defined above.

A method for preparing a compound of formula (II), comprising a step of removing group R¹ from the compound of formula (III) in the presence of a reaction auxiliary to obtain the compound of formula (II), 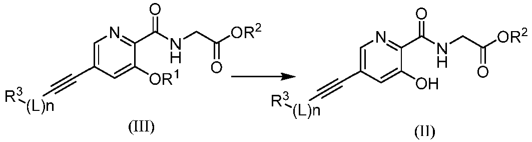 wherein R¹, R², R³, L and n are as defined above.

The method for preparing the compound of formula (II) provided by the present invention is characterized in that the reaction auxiliary is at least one selected from the group consisting of lithium chloride, stannous chloride, stannic chloride, cerium trichloride, antimony pentachloride, ferric chloride, boron trifluoride ethyl ether, boron trichloride and boron tribromide, and preferably lithium chloride; and the reaction solvent is at least one selected from the group consisting of N,N-dimethylformamide, N,N-dimethylacetamide, N-methylpyrrolidone, dimethylsulfoxide, hexamethylphosphoramide and 1,3-dimethyl-2-imidazolinone, and preferably N,N -dimethylacetamide.

The method for preparing the compound of formula (II) provided by the present invention is characterized in that the molar ratio of the reaction auxiliary to the compound of formula (III) is 10:1 to 1:1, and preferably 7:1 to 3:1.

The method for preparing the compound of formula (I) provided by the present invention further comprises the above step for preparing the compound of formula (II).

The method for preparing the compound of formula (I), (II) or (III) provided by the present invention further comprises a step of hydrolysing the compound of formula (II) under an alkaline condition to obtain the compound of formula (I), 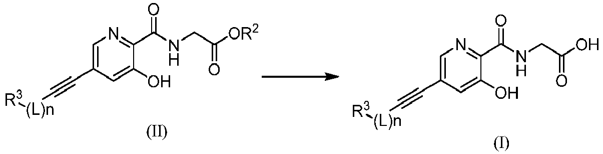 wherein R², R³, L and n are as defined above.

In the method provided by the present invention, in the step of hydrolysing the compound of formula (II) to obtain the compound of formula (I), the alkali is at least one selected from the group consisting of sodium hydroxide, potassium hydroxide, lithium hydroxide, sodium methoxide and sodium ethoxide, and preferably sodium hydroxide.

In the method provided by the present invention, in the step of hydrolysing the compound of formula (II) to obtain the compound of formula (I), the reaction solvent is selected from the group consisting of tetrahydrofuran, N,N-dimethylformamide, N,N-dimethylacetamide, N-methylpyrrolidone, dimethylsulfoxide, hexamethylphosphoramide, 1,3-dimethyl-2-imidazolinone and a mixed solvent thereof with water, and preferably a mixed solvent of N,N-dimethylacetamide/water or a mixed solvent of tetrahydrofuran/water.

The method for preparing the compound of formula (III), (II) or (I) provided by the present invention further comprises a step of obtaining the compound of formula (IV) from a compound of formula (V) in the presence of a condensing agent and solvent,

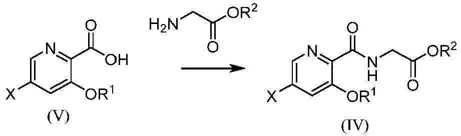

wherein the condensing agent is at least one selected from the group consisting of N-(3-dimethylaminopropyl)-N' -ethylcarbodiimide hydrochloride, dicyclohexylcarbodiimide, N-hydroxysuccinimide, benzotriazol-1-yloxytris(dimethylamino)phosphonium hexafluorophosphate, thionyl chloride, oxalyl chloride, N,N'-carbonyldiimidazole, 1-hydroxybenzotriazole, O-(benzotriazol-1-yl)-N,N,N',N'-tetramethyluronium tetrafluoroborate, 1-hydroxy-7-azobenzotriazole and 1-propylphosphoric anhydride, and preferably N,N' -carbonyldiimidazole;

the solvent is one or more selected from the group consisting of diethyl ether, tetrahydrofuran, isopropanol, dichloromethane, N,N-dimethylformamide, 1,4-dioxane, benzene and toluene, and preferably dichloromethane;

the reaction system optionally comprises an organic alkali, wherein the organic alkali is at least one selected from the group consisting of triethylamine, trimethylamine, diisopropylethylamine, pyridine and p-dimethylaminopyridine; and
wherein X, R¹ and R² are as defined above.

The method for preparing the compound of formula (IV), (III), (II) or (I) provided by the present invention is characterized in that R¹ is a methyl or benzyl; R² is a linear or branched C₁-C₁₀ alkyl; R³ is a halogen-substituted phenyl; L is CH₂; n is 1; and X is bromine.

The method for preparing the compound of formula (IV), (III), (II) or (I) provided by the present invention is characterized in that R¹ is a methyl; R² is a methyl; R³ is a p-chlorophenyl; L is CH₂; n is 1; and X is bromine.

In a preferred embodiment of the present invention, the preparation process for preparing the compounds of formula (IV), (III), (II) and (I) is as follows:

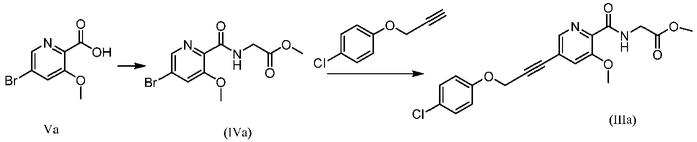

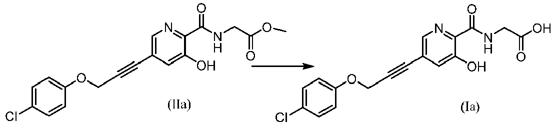

The present invention provides a compound of formula (IVa)

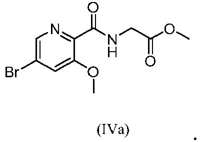

The present invention further provides a method for preparing the compound of formula (IVa) comprising a step of obtaining the compound of formula (IVa) from a compound of formula (Va) in the presence of a condensing agent and solvent, 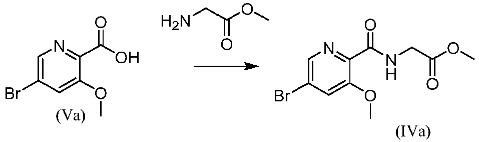
wherein the condensing agent is at least one selected from the group consisting of N-(3-dimethylaminopropyl)-N' -ethylcarbodiimide hydrochloride, dicyclohexylcarbodiimide, N-hydroxysuccinimide, benzotriazol- 1-yloxytris(dimethylamino)phosphonium hexafluorophosphate, thionyl chloride, oxalyl chloride, N,N'-carbonyldiimidazole, 1-hydroxybenzotriazole, O-(benzotriazol-1-yl)-N,N,N',N'-tetramethyluronium tetrafluoroborate, 1-hydroxy-7-azobenzotriazole and 1-propylphosphoric anhydride, and preferably N,N'-carbonyldiimidazole;
the solvent is one or more selected from the group consisting of diethyl ether, tetrahydrofuran, isopropanol, dichloromethane, N,N-dimethylformamide, 1,4-dioxane, benzene and toluene, and preferably dichloromethane;
the reaction system optionally comprises
an organic alkali, wherein the organic alkali is at least one selected from the group consisting of triethylamine, trimethylamine, diisopropylethylamine, pyridine and *p*-dimethylaminopyridine.

The present invention provides a compound of formula (III-A), 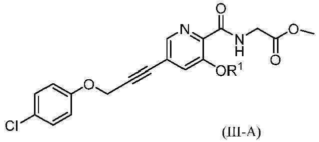 wherein R¹ is as defined above.

The present invention provides a compound of formula (IIIa),

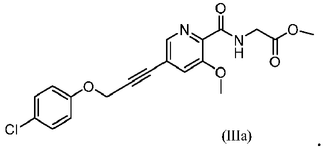

Unless otherwise indicated, the terms used in the present invention have the following meanings:
The hydroxy protecting group of the present invention is a suitable group known in the art for protecting hydroxy. See the hydroxy protecting group described in "Protective Groups in Organic Synthesis", 5^{Th} Ed. T. W. Greene & P. G. M. Wuts. For example, the hydroxy protecting group can preferably be a (C₁₋₁₀ alkyl or aryl)₃silyl group, such as triethylsilyl, triisopropylsilyl, *tert*-butyldimethylsilyl, *tert*-butyldiphenylsilyl, etc. The hydroxy protecting group can be C₁₋₁₀ alkyl or substituted alkyl, preferably alkoxy or aryl-substituted alkyl, more preferably C₁₋₆ alkoxy-substituted C₁₋₆ alkyl or phenyl-substituted C₁₋₆ alkyl, and most preferably C₁₋₄ alkoxy-substituted C₁₋₄ alkyl, for example, methyl, *tert-butyl,* allyl, benzyl, methoxymethyl, ethoxyethyl, 2-tetrahydropyranyl (THP), etc. The hydroxy protecting group can be a (C₁₋₁₀ alkyl or aryl)acyl group, for example, formyl, acetyl, benzoyl, etc. The hydroxy protecting group can be a (C₁₋₆ alkyl or C₆₋₁₀aryl)sulfonyl group. The hydroxy protecting group can also be a (C₁₋₆ alkoxyl or C₆₋₁₀ aryloxy)carbonyl group.
The "carboxy protecting group" is a suitable group known in the art for protecting carboxy. See the carboxy protecting group described in "Protective Groups in Organic Synthesis", 5^{Th} Ed. T. W. Greene & P. G. M. Wuts. For example, the carboxy protecting group is preferably a substituted or unsubstituted linear or branched C₁₋₁₀ alkyl, substituted or unsubstituted linear or branched C₂₋₁₀ alkenyl or alkynyl, substituted or unsubstituted C₃₋₈ cycloalkyl, substituted or unsubstituted C₅₋₁₀ aryl or heteroaryl, or (C₁₋₈ alkyl or aryl)₃silyl group; preferably a linear or branched C₁₋₆ alkyl, and more preferably a linear or branched C₁₋₄ alkyl.

| Pd₂(dba)₃ | Tris(dibenzylideneaceto ne)dipalladium | Pd(dba)₂ | Bis(dibenzylideneacetone)palladium |
|---|---|---|---|
| Pd(OAc)₂ | Palladium acetate | Pd(OCOCF₃) | Palladium trifluoroacetate |
| Pd(Piv)₂ | Palladium trimethylacetate | Pd(OTf)₂ | Palladium trifluoromethanesulfonate |
| Pd(PPh₃)₄ | Tetrakis(triphenylphosph ine)palladium | PdCl₂ | Palladium chloride |
| Pd(PPh₃)₂Cl₂ | Dichlorobis(triphenylph osphine)palladium | Pd(dppf)Cl₂ | [1,1'-Bis(diphenylphosphino)ferrocene ]palladium dichloride |

### DETAILED DESCRIPTION OF THE INVENTION

The present invention will be illustrated in more detail below in combination with the examples. The examples of the present invention are only used to illustrate the technical solutions of the present invention, and the essence and scope of the present invention are not limited thereto.

The compound of formula (Va) was prepared according to the method in patent application WO2010018458A, and the compound of formula (Vc) was prepared according to the method in patent application CN104276999A.

### Example 1

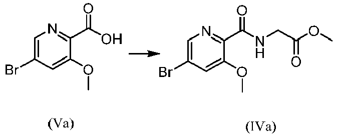

Compound Va (650 g) and dichloromethane (6.9 kg) were added to a 20 L reactor successively, then N,N'-carbonyldiimidazole (500 g) was slowly added at room temperature. After completion of the addtion, the reaction solution was stirred at room temperature for one hour, then glycine methyl ester hydrochloride (387 g) was slowly added. After HPLC showed that the reaction was complete, the reaction solution was extracted with water. The organic phase was concentrated under reduced pressure, and *n*-heptane (3.536 kg) was added dropwise at room temperature. The solution was filtered, and the filter cake was dried to obtain the product (719 g), purity: 98.5%, yield: 84%.

### Example 2

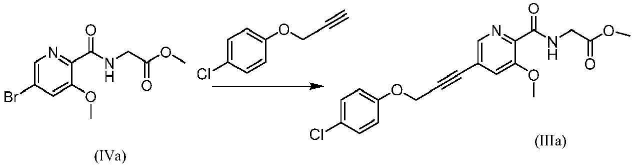

Compound IVa (600 g), tetrahydrofuran (1.6 kg), triethylamine (400 g), cuprous iodide (7.5 g) and dichlorobis(triphenylphosphine)palladium (27.8 g) were successively added to a 10 L reactor under a nitrogen atmosphere. The reaction solution was warmed up to about 65°C, then a solution of 3-*p*-chlorophenoxypropyne (495 g) in tetrahydrofuran (534 g) was slowly added dropwise. The reaction solution was stirred at the same temperature until HPLC showed that the reaction was complete. The reaction solution was concentrated under reduced pressure to remove tetrahydrofuran, then ethyl acetate (4.32 kg), water (1.2 kg) and 1 wt% aqueous hydrochloric acid solution (1.5 kg) were successively added to the residual solution to adjust pH to 2-3. Water (0.9 kg) was added, and the solution was left to stand for phase separation. The aqueous phase was extracted with ethyl acetate. The combined organic phase was washed with water and filtered. The filtrate was concentrated under reduced pressure, then methyl *tert-butyl* ether (4.44 kg) and *n*-heptane (1.224 kg) were successively added dropwise. The solution was stirred at room temperature overnight, filtered, and the filter cake was dried to obtain a brown solid (672 g), yield: 87%, purity: 98.8%.

### Example 3

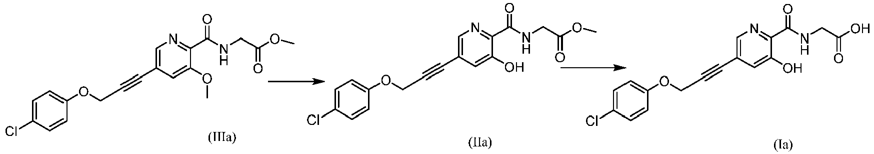

N,N-Dimethylacetamide (2.8 kg), compound IIIa (600 g) and anhydrous lithium chloride (327 g) were successively added to a 20 L reactor under a nitrogen atmosphere, and the reaction solution was warmed up to 80-130°C to react. After HPLC showed that the reaction was complete, the reaction solution was cooled to 50°C, and 724 g 17 wt% aqueous sodium hydroxide solution was slowly added dropwise. After completion of the hydrolysis reaction, the reaction solution was cooled to room temperature, then water (600 g) and ethyl acetate (13.5 kg) were successively added. Concentrated hydrochloric acid (1.56 kg) was slowly added dropwise, and the reaction solution was stirred for one hour and filtered. The two phases were separated, and the organic phase was concentrated under reduced pressure. Isopropanol (7 kg) and water (3.6 kg) were added to the residual solution at room temperature. The solution was suction-filtered, and the filter cake was dried to obtain the product Ia (408 g), purity: 96.5%, yield: 71%.

### Example 4

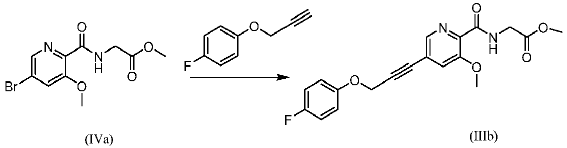

The above step was carried out in accordance with Example 2, wherein 3-*p*-chlorophenoxypropyne was replaced with 3-*p*-fluorophenoxypropyne. Compound IVa (15 g), tetrahydrofuran (45 mL), triethylamine (10 g), cuprous iodide (0.15 g) and dichlorobis(triphenylphosphine)palladium (0.556 g) were successively added to a reaction flask under a nitrogen atmosphere. The reaction solution was warmed up to about 65°C, then a solution of 3-*p*-fluorophenoxypropyne (12.6 g) in tetrahydrofuran (36 mL) was slowly added dropwise. The reaction solution was stirred at the same temperature until HPLC showed that the reaction was complete. Ethyl acetate (75 mL) was added, then the reaction solution was filtered. Water and 6 M hydrochloric acid were added to the filtrate to adjust the pH to 3-5, and the two phases were separated. The aqueous phase was extracted with ethyl acetate. The combined organic phase was washed with water, dried over anhydrous sodium sulfate, and concentrated to dryness under reduced pressure. Ethyl acetate (30 mL) was added to the residue, and the solution was heated to reflux until it was clear. Methyl *tert-butyl* ether (115 mL) was added dropwise at 35°C, and the solution was cooled to 0°C and stirred overnight. The solution was filtered, and the filter cake was dried to obtain an off-white solid (10.1 g), yield: 55%, purity: 98.3%.

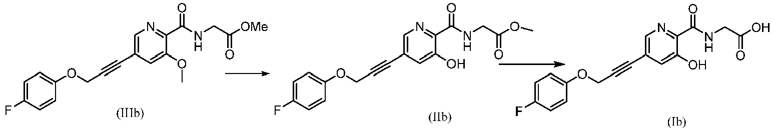

The above steps were carried out in accordance with Example 3. Compound IIIb (25 g), N,N-dimethylacetamide (125 mL) and anhydrous lithium chloride (17.1 g) were successively added to a reaction flask under a nitrogen atmosphere, and the reaction solution was warmed up to 105-110°C to react. After HPLC showed that the reaction was complete, the reaction solution was cooled to 50°C, and 33.4 g 16 wt% aqueous sodium hydroxide solution was slowly added dropwise. After completion of the hydrolysis reaction, the reaction solution was cooled to room temperature, then water (250 g) and ethyl acetate (625 mL) were successively added. Concentrated hydrochloric acid (56 mL) was slowly added dropwise, and the two phases were separated. The organic phase was washed with water, and concentrated under reduced pressure. Acetonitrile (40 mL) and water (150 mL) were added to the residual solution at room temperature. The solution was suction-filtered, and the filter cake was dried to obtain an off-white solid compound Ib (16.7 g), two-step yield: 72%, purity: 98.6%.

### Example 5

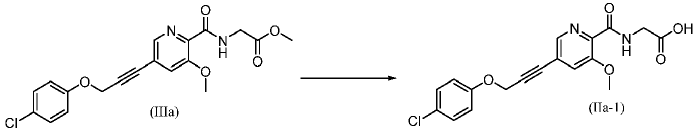

Compound IIIa (5 g) was dissolved in tetrahydrofuran/water (25/5 mL) followed by the addition of lithium hydroxide (0.63 g), and the reaction was carried out at room temperature. After completion of the reaction, ethyl acetate (200 mL), water (50 mL) and concentrated hydrochloric acid (10 mL) were successively added. The organic phase was washed with water, dried over anhydrous sodium sulfate, and concentrated under reduced pressure. The resulting residue was pulped in methyl *tert-butyl* ether, suction-filtered, and the filter cake was dried to obtain the product IIa-1 (4.0 g), yield: 83%, purity: 97.5%.

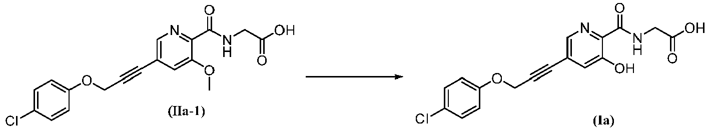

Compound IIa-1 (2 g), anhydrous lithium chloride (1.2 g) and N,N-dimethylacetamide (10 mL) were successively added to a reaction flask under a nitrogen atmosphere, and the reaction was carried out at 90°C until completion. The reaction solution was cooled, then water (20 g) and ethyl acetate (50 mL) were successively added. Concentrated hydrochloric acid was slowly added dropwise to adjust pH to 1, and the two phases were separated. The organic phase was dried and concentrated under reduced pressure. Acetonitrile (5 mL) and water (20 g) were added to the residual solution. The solution was suction-filtered, and the filter cake was dried to obtain the product Ia (1.5 g), purity: 83%, yield: 78%.

**HPLC results of different reaction temperature**

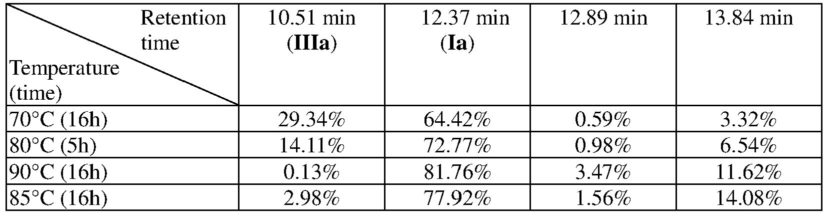

In the above table, 70°C, 80°C and 90°C refer to the results of continuous reaction at stepped-up temperature in the same batch. It can be seen that with the increase of the reaction temperature, the raw materials will react more completely, but the impurities will increase, which is difficult to remove in the post-treatment. The two-step yield of the new route is 65% in total, and the purity of the resulting product is low.

### Example 6

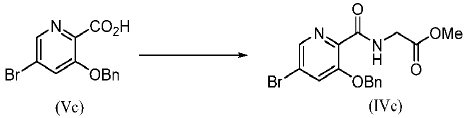

Intermediate Vc (20 g) was dissolved in 400 mL of dichloromethane, and triethylamine (27 mL) and 1-hydroxybenzotriazole (12.0 g) were added successively. After the reaction solution was stirred for 10 minutes, N-(3-dimethylaminopropyl)-N'-ethylcarbodiimide hydrochloride (16.5 g) and glycine methyl ester hydrochloride (8.64 g) were added. The reaction solution was reacted at room temperature for 6 hours. After completion of the reaction, the reaction solution was washed with saturated sodium bicarbonate, water and saturated brine successively, dried with anhydrous sodium sulfate and concentrated under reduced pressure. The crude product was purified by silica gel column chromatography. This step was repeated 5 times, and this step was also repeated once with 18 g of intermediate Vc. The products were combined to obtain a total of 85.5 g of white solid compound (85.5 g), yield: 58.9%.

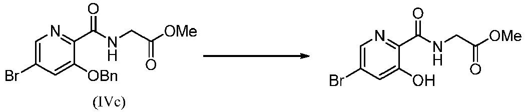

Intermediate IVc (25.0 g) was dissolved in 500 mL of dichloromethane, and 70 mL of boron trifluoride ethyl ether was added to the reaction flask. The reaction solution was heated to 45°C and reacted for 6 hours. After completion of the reaction, 300 mL of saturated ammonium chloride solution was added, and the reaction solution was then stirred for two hours. The two phases were separated. The organic phase was washed with water and saturated brine successively, and concentrated under reduced pressure to remove the organic solvent and obtain a crude product. This step was repeated 3 times, and this step was also repeated once with 10.5 g of intermediate IVc. The resulting crude products were combined, and then recrystallized from dichloromethane to obtain a pale yellow solid product (34.4 g), yield: 52.7%.

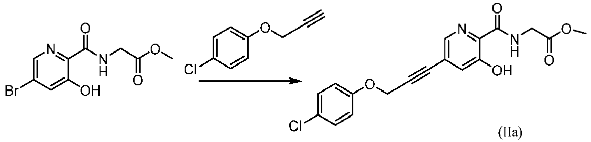

Compound methyl N-(5-bromo-3-hydroxy-pyridine-2-formyl)glycinate (4.0 g), 3-*p*-chlorophenoxypropyne (3.2 mL), triethylamine (5 mL), dichlorobis(triphenylphosphine)palladium (700 mg), cuprous iodide (700 mg) and N,N-dimethylformamide (5 mL) were added to a microwave tube. The microwave tube was placed into a CEM microwave reactor and reacted for 20 minutes at a microwave power of 300 W and a temperature of 120°C. At the same time, this reaction can also be carried out by heating to 80°C for 8 to 10 hours. The reactor was cooled to room temperature with air flow. The reaction mixture was diluted with dichloromethane and filtered through celite, and the filter cake was washed with dichloromethane. The organic phase was washed with water and saturated brine, and dried with anhydrous sodium sulfate. This step was repeated 10 times. The products were combined and then purified by column chromatography to obtain a white solid product (27.5 g), yield: 52.8%.

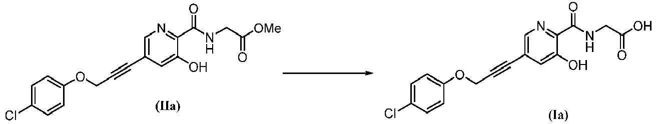

Compound IIa (27.0 g) was dissolved in 10% aqueous tetrahydrofuran solution. Lithium hydroxide was added, and the reaction solution was then heated to 35°C for reaction. After completion of the reaction, the reaction solution was concentrated, and an appropriate amount of water was added to dissolve the resulting residue completely. 10% dilute hydrochloric acid was added to neutralize the reaction solution until the solid was completely precipitated. The solid was suction-filtered and dried to obtain the product Ia (20.5 g), yield: 78.9%.

## Claims

1. A method for preparing a compound of formula (III), **characterized in that** a compound of formula (IV) is reacted with a compound of formula (VI) in the presence of a palladium catalyst, copper catalyst and alkali to obtain the compound of formula (III), 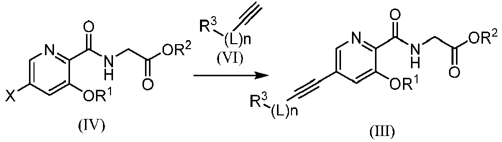
wherein R¹ is a hydroxy protecting group;
R² is a carboxy protecting group;
R³ is a C₁-C₄ alkyl, phenyl, substituted phenyl, 5 to 6 membered heteroaryl containing oxygen or nitrogen, substituted 5 to 6 membered heteroaryl containing oxygen or nitrogen, wherein the substituent is a C₁-C₄ alkyl, C₁-C₄ alkoxy, C₁-C₄ haloalkyl, halogen, cyano, 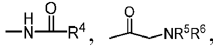 phenyl, or 5 to 6 membered heteroaryl containing oxygen or nitrogen, wherein R⁴ is a C₁-C₄ alkyl; R⁵ and R⁶ are each independently selected from the group consisting of hydrogen and C₁-C₄ alkyl, or R⁵ and R⁶ are connected to form a 3 to 7 membered heterocyclyl containing nitrogen;
L is -CH₂-, -CH₂O- or 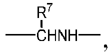 wherein R⁷ is selected from the group consisting of hydrogen, C₁-C₄ alkyl and phenyl;
n is 0 or 1; and
X is an iodine, bromine, chlorine, or triflate group.

2. The method according to claim 1, **characterized in that** the palladium catalyst is at least one selected from the group consisting of Pd₂(dba)₃, Pd(dba)₂, Pd(OAc)₂, Pd(tfa)₂, Pd(Piv)₂, Pd(OTf)₂, Pd(PPh₃)₄, PdCl₂, Pd(PPh₃)₂Cl₂ and Pd(dppf)Cl₂, and preferably Pd(PPh₃)₂Cl₂; the copper catalyst is at least one selected from the group consisting of CuI, CuBr, CuCl and CuF, and preferably CuI; and the alkali is at least one selected from the group consisting of triethylamine, trimethylamine and diisopropylethylamine, and preferably triethylamine.

3. The method according to claim 1, **characterized in that** the molar ratio of the palladium catalyst to the compound of formula (IV) is 0.001:1 to 1:1, and preferably 0.01:1 to 0.05:1; and the molar ratio of the copper catalyst to the palladium catalyst is 10:1 to 1:10, and preferably 2:1 to 1:2.

4. The method according to claim 1, **characterized in that** the reaction solvent is at least one selected from the group consisting of tetrahydrofuran, 2-methyltetrahydrofuran, 1,4-dioxane, N,N-dimethylformamide, N,N-dimethylacetamide, N-methylpyrrolidone, dimethyl sulfoxide, hexamethylphosphoramide and 1,3-dimethyl-2-imidazolinone, and preferably tetrahydrofuran.

5. A method for preparing a compound of formula (I), **characterized by** comprising the method for preparing the compound of formula (III) according to any one of claims 1 to 4, and a further step of removing groups R¹ and R², 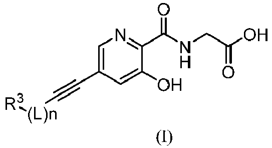 wherein R¹, R², R³, L and n are as defined in claim 1.

6. A method for preparing a compound of formula (II), **characterized by** comprising a step of removing group R¹ from a compound of formula (III) in the presence of a reaction auxiliary, 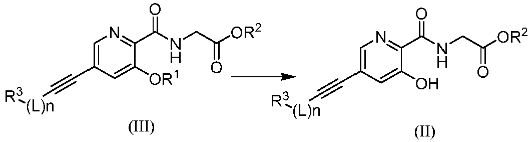 wherein R¹, R², R³, L and n are as defined in claim 1.

7. The method according to claim 6, **characterized in that** the reaction auxiliary is at least one selected from the group consisting of lithium chloride, stannous chloride, stannic chloride, cerium trichloride, antimony pentachloride, ferric chloride, boron trifluoride ethyl ether, boron trichloride and boron tribromide, and preferably lithium chloride; and the reaction solvent is at least one selected from the group consisting of N,N-dimethylformamide, N,N-dimethylacetamide, N-methylpyrrolidone, dimethylsulfoxide, hexamethylphosphoramide and 1,3-dimethyl-2-imidazolinone, and preferably N,N-dimethylacetamide.

8. The method according to claim 7, **characterized in that** the molar ratio of the reaction auxiliary to the compound of formula (III) is 10:1 to 1:1, and preferably 7:1 to 3:1.

9. The method for preparing the compound of formula (I) according to claim 5, **characterized by** further comprising the step for preparing the compound of formula (II) according to any one of claims 6 to 8.

10. The method according to any one of claims 5 to 9, further comprising a step of hydrolysing the compound of formula (II) under an alkaline condition to obtain the compound of formula (I), 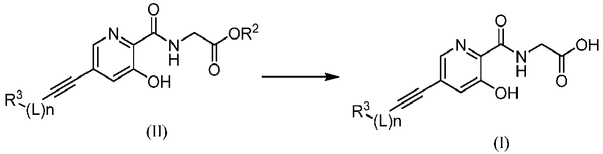 wherein R², R³, L and n are as defined in claim 1.

11. The method according to claim 10, **characterized in that** the alkali is at least one selected from the group consisting of sodium hydroxide, potassium hydroxide, lithium hydroxide, sodium methoxide and sodium ethoxide, and preferably sodium hydroxide.

12. The method according to any one of claims 1 to 11, **characterized by** further comprising a step of obtaining the compound of formula (IV) from a compound of formula (V) in the presence of a condensing agent and solvent, 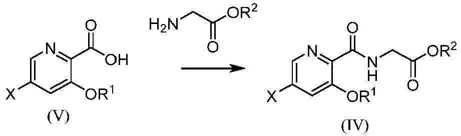
wherein the condensing agent is at least one selected from the group consisting of N-(3-dimethylaminopropyl)-N' -ethylcarbodiimide hydrochloride, dicyclohexylcarbodiimide, N-hydroxysuccinimide, benzotriazol- 1-yloxytris(dimethylamino)phosphonium hexafluorophosphate, thionyl chloride, oxalyl chloride, N,N'-carbonyldiimidazole, 1-hydroxybenzotriazole, O-(benzotriazol-1-yl)-N,N,N',N'-tetramethyluronium tetrafluoroborate, 1-hydroxy-7-azobenzotriazole and 1-propylphosphoric anhydride, and preferably N,N' -carbonyldiimidazole;
the solvent is one or more selected from the group consisting of diethyl ether, tetrahydrofuran, isopropanol, dichloromethane, N,N-dimethylformamide, 1,4-dioxane, benzene and toluene, and preferably dichloromethane;
the reaction system optionally comprises an organic alkali, wherein the organic base is selected from the group consisting of triethylamine, trimethylamine, diisopropylethylamine, pyridine and *p*-dimethylaminopyridine; and
wherein X, R¹ and R² are as defined in claim 1.

13. The method according to any one of claims 1 to 12, **characterized in that** R¹ is a methyl or benzyl; R² is a linear or branched C₁-C₁₀ alkyl; R³ is a halogen-substituted phenyl; L is CH₂; n is 1; and X is bromine.

14. The method according to any one of claims 1 to 12, **characterized in that** R¹ is a methyl; R² is a methyl; R³ is a p-chlorophenyl; L is CH₂; n is 1; and X is bromine.

15. The method according to any one of claims 1 to 14, comprising the following
steps of

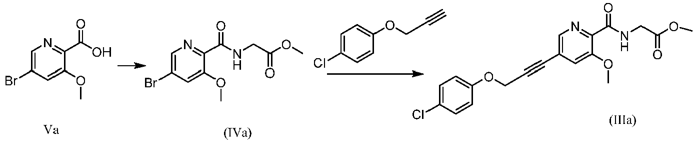

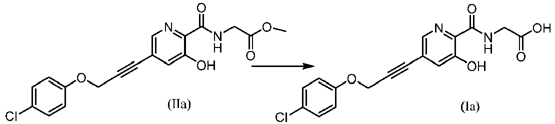

16. A compound of formula (IVa),

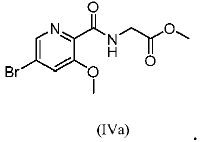

17. A method for preparing a compound of formula (IVa), **characterized by** comprising a step of obtaining the compound of formula (IVa) from a compound of formula (Va) in the presence of a condensing agent and solvent, 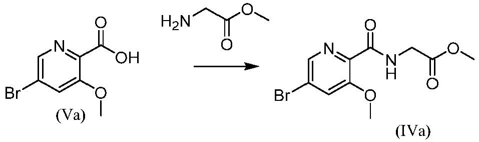
wherein the condensing agent is at least one selected from the group consisting of N-(3-dimethylaminopropyl)-N' -ethylcarbodiimide hydrochloride, dicyclohexylcarbodiimide, N-hydroxysuccinimide, benzotriazol- 1-yloxytris(dimethylamino)phosphonium hexafluorophosphate, thionyl chloride, oxalyl chloride, N,N'-carbonyldiimidazole, 1-hydroxybenzotriazole, O-(benzotriazol-1-yl)-N,N,N',N'-tetramethyluronium tetrafluoroborate, 1-hydroxy-7-azobenzotriazole and 1-propylphosphoric anhydride, and preferably N,N' -carbonyldiimidazole;
the solvent is one or more selected from the group consisting of diethyl ether, tetrahydrofuran, isopropanol, dichloromethane, N,N-dimethylformamide, 1,4-dioxane, benzene and toluene, and preferably dichloromethane;
the reaction system optionally comprises an organic alkali, wherein the organic alkali is at least one selected from the group consisting of triethylamine, trimethylamine, diisopropylethylamine, pyridine and *p*-dimethylaminopyridine.

18. A compound of formula (III-A), 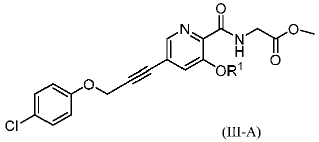 wherein R¹ is as defined in claim 1.

19. A compound of formula (IIIa),
